# EUROPEAN PATENT SPECIFICATION

(11) **EP 1 989 942 B1**
(45) Date of publication and mention of the grant of the patent: **15.08.2012**
(21) Application number: 07831075.2
(22) Date of filing: 01.11.2007
(51) Int. Cl.: A23C 9/123, C12N 1/20

(54) **METHOD OF PRODUCING FERMENTED MILK USING NOVEL LACTIC ACID BACTERIUM**
VERFAHREN ZUR HERSTELLUNG FERMENTIERTER MILCH MITHILFE EINES NEUARTIGEN MILCHSÄUREBAKTERIUMS
PROCÉDÉ DE PRODUCTION DE LAIT FERMENTÉ À L'AIDE D'UNE NOUVELLE BACTÉRIE D'ACIDE LACTIQUE

(30) Priority: 13.02.2007 JP 2007032646
(43) Date of publication of application: 12.11.2008
(73) Proprietor: Morinaga Milk Industry Co., Ltd., Minato-ku Tokyo 108-8384 (JP)
(72) Inventor: SHIMIZU, Kanetada, Zama-shi Kanagawa 228-8583 (JP); MIYAJI, Kazuhiro, Zama-shi Kanagawa 228-8583 (JP); OGAWA, Kouichi, Zama-shi Kanagawa 228-8583 (JP); KISO, Yoshiaki, Zama-shi Kanagawa 228-8583 (JP); ISHIDA, Takako, Zama-shi Kanagawa 228-8583 (JP)
(74) Representative: Ilgart, Jean-Christophe
(86) International application number: PCT/JP2007/071341
(87) International publication number: WO 2008/099543

(56) References cited:
- WO-A1-98/35564
- JP-A- 2002 335 860

## Description

### TECHNICAL FIELD

The present invention relates to a method for producing fermented milk using novel lactic acid bacteria belonging to the genus *Lactococcus,* and fermented milk prepared in accordance with the production method.
Priority is claimed on Japanese Patent Application No. 2007-032646, filed on February 13, 2007, the contents of which are incorporated herein by reference.

### BACKGROUND ART

Bacteria belonging to the genus *Bifidobacterium* (hereinafter, abbreviated to "*Bifidobacterium*"), such as *Bifidobacterium longum,* is one of predominant bacterial strains in intestinal microflora formed in the human intestinal tract. It is known that *Bifidobacterium* have an intestinal function-regulating activity, an immuno-stimulating activity, and an anti-cancer activity. Accordingly, demands for food products containing viable *Bifidobacterium* such as milk fermented with *Bifidobacterium* or the like are increasing in accordance with an increase of health consciousness of consumers.

*Bifidobacterium* exhibits a poor proliferation potency in a milk medium. Accordingly, various growth-stimulating substances are generally formulated in fermented milk so that the *Bifidobacterium* is contained therein at a constant content, for example, 1 × 10⁷ CFU/ml. However, the growth-stimulating substances are generally expensive and may degrade the taste. In addition, preservation of *Bifidobacterium* under acidic conditions is difficult and tends to result in death of *Bifidobacterium.* Thus, the viable count of the *Bifidobacterium* in fermented milk products decreases with accelerating speed during the course of distribution of the fermented milk products.
Accordingly, it is expected that a promotion of the growth of *Bifidobacterium* or an improvement of the survivability thereof during storage enables not only preparation of fermented milk containing a large amount of viable *Bifidobacterium,* but also preparation of fermented milk which can keep an abundant amount of viable *Bifidobacterium* from immediately after preparation until being consumed.

Various methods for promoting the growth of *Bifidobacterium* or improving the survivability thereof during storage by fermentation with *Bifidobacterium* and another lactic acid bacterium without adding any growth-stimulating substances or the like have been disclosed. For example, (1) yogurt containing *Lactococcus lactis subsp. lactis, Lactococcus lactis subsp. cremoris,* and *Bifidobacterium,* and a method for preparing the yogurt have been disclosed (see, for example, Patent Document 1), with respect to a method for promoting the growth *of Bifidobacterium* to prepare fermented milk.

For example, (2) a method for fermenting milk with *Bifidobacterium,* including cultivating *Bifidobacterium breve* together with *Lactococcus lactis subsp. lactis,* which forms neither diacetyl nor acetoin, in a medium containing milk as the main component thereof has been disclosed (see, for example, Patent Document 2), with respect to a method for improving the survivability of *Bifidobacterium* during storage of fermented milk.
Patent Document 1: Japanese Patent Publication No. 3,364,491.
Patent Document 2: Japanese Patent Publication No. 3,068,484.
FR 2842707 discloses the preparation of yogurt using a co-culture of L. lactis and Bifidobacterium animalis

### DISCLOSURE OF THE INVENTION

### [Problems to be Solved by the Invention]

Although the growth of *Bifidobacterium* is promoted and the fermentation time is shortened in accordance with the above-mentioned method (1), there is no disclosure with respect to the survivability of *Bifidobacterium* during storage in Patent Document 1. In contrast, although both growth-stimulating effects and survivability-improving effects are recognized by using a mixture composed of a specific *Bifidobacterium* and a specific lactic acid bacterium in accordance with the above-mentioned method (2), there is no disclosure with respect to *Bifidobacterium* other than *Bifidobacterium breve,* such as *Bifidobacterium longum,* which is generally formulated in food products.

The present invention has for an object thereof the provision of a method for producing fermented milk using lactic acid bacteria which stimulate the growth of *Bifidobacterium* and improve the survivability thereof during storage, and fermented milk prepared in accordance with the production method.

### [Means for Solving the Problems]

The inventors of the present invention intensively investigated so as to solve the above-mentioned problems, and performed a fermentation test by performing co-cultivation with *Bifidobacterium longum* to find out lactic acid bacterial strains which exhibit excellent fermentability in 10% (W/W) reconstituted skim milk medium. As a result, the inventors found lactic acid bacterium strains which can promote the growth of *Bifidobacterium longum* to a viable count of 5 × 10⁸ CFU/g when pH is 4.4 to 4.6, and enhance the survival rate of *Bifidobacterium longum* to 30% or more when stored at 10°C for two weeks after the fermentation is ended and then immediately rapid cooling is performed. In addition, the present inventors found that fermented milk containing a great amount of *Bifidobacterium* and having an excellent survivability during storage can be produced by using the lactic acid bacteria, and thus the present invention has been completed.

That is, the present invention provides a method for producing fermented milk including: performing fermentation using, as lactic acid bacteria, both bacteria belonging to the genus *Bifidobacterium* and bacteria belonging to the genus *Lactococcus* having the following bacteriological properties:
(1) fermentability which curdles a 10% (W/W) reconstituted skim milk medium when cultivated at a temperature of 25°C to 37°C for 16 hours;
(2) *Bifidobacterium longum* growth-promoting properties which lead to a viable count of *Bifidobacterium longum* of 5×10⁸ CFU/g or more, when co-cultivated with *Bifidobacterium longum* in the 10% (W/W) reconstituted skim milk medium until the pH thereof is 4.4 to 4.6; and
(3) *Bifidobacterium longum* survivability-improving properties during storage, which lead to a survival rate of *Bifidobacterium longum* of 30% or more, after co-cultivation with *Bifidobacterium longum* in the 10% (W/W) reconstituted skim milk medium until the pH thereof is 4.4 to 4.6, rapid cooling, and two weeks storage at 10°C.
The present invention also provides a method for producing fermented milk, characterized in that the bacteria belonging to the genus *Lactococcus* have no ability to ferment xylose and produce neither diacetyl nor acetoin.
The present invention also provides a method for producing fermented milk, characterized in that the bacteria belonging to the genus *Lactococcus* are *Lactococcus lactis* subsp. *lactis.*
The present invention also provides a method for producing fermented milk, characterized in that the bacteria belonging to the genus *Lactococcus* include at least one bacterial strain selected from the group consisting of *Lactococcus lactis subsp. lactis* MCC852 (FERM BP-10742), *Lactococcus lactis subsp. lactis* MCC857 (FERM BP-10757), *Lactococcus lactis subsp. lactis* MCC859 (FERM BP-10744), *Lactococcus lactis subsp. lactis* MCC865 (FERM BP-10745), and *Lactococcus lactis subsp. lactis* MCC866 (FERM BP-10746).
The present invention also provides a method for producing fermented milk, characterized in that the bacteria belonging to the genus *Bifidobacterium* are *Bifidobacterium longum.*
The present invention also provides a method for producing fermented milk, characterized in that a bacterial strain of the *Bifidobacterium longum* is *Bifidobacterium longum* FERM BP-7787.
The present invention also provides a method for producing fermented milk, characterized in that both *Streptococcus thermophilus* and *Lactobacillus bulgaricus* are further used as the lactic acid bacteria.
The present invention also provides fermented milk prepared by any of the above-mentioned method for producing fermented milk.

### [Effects of the Invention]

In accordance with the method for producing fermented milk according to the present invention, fermented milk containing a great amount of *Bifidobacterium,* particularly *Bifidobacterium longum* can be efficiently produced as never before. In addition, the fermented milk according to the present invention maintains a sufficient viable count of *Bifidobacterium,* particularly *Bifidobacterium longum,* even during the course of distribution, and therefore the fermented milk exhibit further high intestinal function-regulating effects and is useful for health control.

### BEST MODE FOR CARRYING OUT THE INVENTION

The present invention relates to a method for producing fermented milk, characterized in that milk is fermented with both *Bifidobacterium* and bacteria belonging to the genus *Lactococcus* having the above-mentioned properties (1), (2), and (3). In particular, the method is suitable for production of fermented milk by fermenting milk using *Bifidobacterium longum.*

Examples of *Bifidobacterium longum* available in the present invention include *Bifidobacterium longum* FERM BP-7787, *Bifidobacterium longum-*type strain ATCC 15707, and the like. *Bifidobacterium longum* FERM BP-7787 is particularly preferable, since *Bifidobacterium longum* FERM BP-7787 exhibits excellent fermentability in a milk medium, excellent acid-resistance during the course of distribution, and excellent gastric acid-resistance. *Bifidobacterium longum* FERM BP-7787 was accepted by the International Patent Organism Depositary, National Institute of Advanced Industrial Science and Technology, (Central 6, 1-1, Higashi 1-Chome Tsukuba-shi, Ibaraki-ken, JAPAN (postal code number: 305-8566)) on October 31, 2001.

The bacteria belonging to the genus *Lactococcus* to be used in the present invention have the properties (1), (2), and (3).
The property (1) relates to fermentability. If a lactic acid bacteria can rapidly proliferate and have a strong fermentability sufficient to curdle a 10% (W/W) reconstituted skim milk medium when cultivated therein at a temperature between 25°C and 37°C for 16 hours, the lactic acid bacteria can effectively promote the growth of *Bifidobacterium,* particularly *Bifidobacterium longum,* at the time of preparing fermented milk. As used herein, the phrase "curdle a culture medium" refers to a phenomenon in which the pH of the culture medium decreases below an isoelectric point of a milk protein thereof by acid fermentation, and thereby the milk protein agglomerates and the culture medium is curdled. The "10% (W/W) reconstituted skim milk medium" may be prepared, for example, by dissolving 10% by mass of skim milk powders (manufactured by Morinaga Milk Industry Co., Ltd., for example) in water.

Although the temperature range suitable for fermentation with bacteria belonging to the genus *Lactococcus* is generally between 20°C and 30°C, the bacteria belonging to the genus *Lactococcus* to be used in the present invention exhibit a strong fermentability at a temperature between 25°C and 37°C. In other words, the bacteria belonging to the genus *Lactococcus* to be used in the present invention exhibit a sufficient fermentability within a temperature range suitable for fermentation with *Bifidobacterium,* particularly with *Bifidobacterium longum* (30°C to 40°C).

The property (2) relates to *Bifidobacterium longum* growth-promoting properties. A milk medium such as a 10% (W/W) reconstituted skim milk medium exhibits excellent taste, mouth-feeling, and external appearance, when the pH thereof reaches approximately 4.6, casein and other components contained therein are generally precipitated, and the culture medium is wholly curdled. Accordingly, fermentation is generally stopped by rapid cooling or the like when the pH reaches approximately 4.6, to prepare fermented milk products. Therefore, the lactic acid bacteria having the growth-promoting properties that can lead the viable count of *Bifidobacter-ium longum* being a high count of 5×10⁸ CFU/g or more when co-cultivated with *Bifidobacterium longum* in the 10% (W/W) reconstituted skim milk medium until pH thereof is 4.4 to 4.6 can effectively increase the viable count of *Bifidobacterium,* particularly *Bifidobacterium longum,* in fermented milk at the time of preparation of fermented milk.

The property (3) relates to *Bifidobacterium longum* survivability-improving properties during storage. The quality preservation time period of fermented milk products is generally about two weeks when stored at 10°C or lower. Accordingly, fermented milk which maintains a sufficient viable count of *Bifidobacterium,* particularly *Bifidobacterium longum,* even at the end of the time period by which the quality preservation thereof is ensured, can be produced, provided that the lactic acid bacteria having the survivability-improving properties during storage that can lead to a survival rate of *Bifidobacterium longum* being 30% or more after co-cultivation with *Bifidobacterium longum* in the 10% (W/W) reconstituted skim milk medium until the pH thereof is 4.4 to 4.6, rapid cooling, and two weeks storage at 10°C.

The bacteria belonging to the genus *Lactococcus* to be used in the present invention may be prepared in accordance with the following method, for example. First, bacterial strains are isolated from various samples, and strains which exhibit excellent fermentability in the 10% (W/W) reconstituted skim milk medium, more specifically fermentability sufficient to curdle the 10% (W/W) reconstituted skim milk medium when cultivated therein at a temperature of 25 to 37°C for 16 hours, are selected from the isolated bacterial strains. Then, the selected bacterial strains are co-cultivated with *Bifidobacterium longum,* and bacterial strains which have *Bifidobacterium longum* growth-promoting properties and *Bifidobacterium longum* survivability-improving properties during storage defined as the above-mentioned properties (2) and (3) are selected. It is further preferable that bacterial strains which do not have an ability to ferment xylose and produce neither diacetyl nor acetoin be selected.

In the following, the present invention will be explained in more detail.

### 1. Isolation of bacterial strains

In order to isolate bacterial strains having the above-mentioned properties from the natural world, the present inventors collected samples from the natural world in Japan, diluted the samples with an anaerobic dilution buffer ("The World of Enterobacteria" published by Soubunsha Co., Ltd., written by Tomotari Mitsuoka, Page 322, 1980: hereinafter, abbreviated to "Reference 1"), inoculated the diluted samples on each plate of Briggs liver broth (see the above-mentioned Reference 1, Page 319), and then cultured the inoculated samples at 30°C under anaerobic conditions. Among the thus obtained colonies, bacterial strains which showed morphological characteristics of streptococcal bacteria and were recognized as Gram-positive bacteria by microscopic observation of applied specimens were picked up. The picked up strains were each streak-inoculated on each BL agar flat plate and then repeatedly cultivated under anaerobic conditions in the same manner as described above to obtain purely isolated bacterial strains. The isolated bacterial strains were subjected to a fermentation test on a 10% (W/W) reconstituted skim milk medium as described below to obtain 20 bacterial strains with fermentability defined as the above-mentioned property (1). Then, the obtained bacterial strains were co-cultured with *Bifidobacterium longum* to obtain 5 bacterial strains having both the growth-promoting properties which raise the viable count of *Bifidobacterium longumi* at a pH of 4.4 to 4.6 to 5×10⁸ CFU/g or more and the survivability-improving properties during storage which raise the survival rate of *Bifidobacterium longum* to 30% or more when stored at 10°C for two weeks after rapid cooling at a pH of 4.4 to 4.6. The 5 bacterial strains are named as "MCC852", "MCC857", "MCC859", "MCC865", and "MCC866", respectively.

### 2. Bacteriological properties

The bacteriological properties of the 5 bacterial strains will be shown in the following. The tests for determining the bacteriological properties were performed with reference to Bergey's Manual of Systematic Bacteriology, edited by Peter H. A. Sneath, Vol. 2, published by Williams and Wilkins Company, 1986).

(I) Bacterial morphology (observed through an optical microscope after anaerobic cultivation on a BL agar flat plate at 30°C for 72 hours)
   Size: 1 to 2 µm (diameter)
   Morphology: Streptococcal bacteria
(II) Gram staining: Positive
(III) Litmus milk: Curdled
(IV) Endospore formation: Negative
(V) Gas production from glucose: Negative
(VI) Motility: Negative
(VII) Catalase activity: Negative
(VIII) Arginine decarboxylase test: Positive
(IX) Gas production from citric acid: Negative
(X) Temperature susceptibility (at 60°C for 30 minutes and at 65°C for 30 minutes): Positive in both cases
(XI) Glucose degradation product: L-lactic acid

**Table 1.**

| | Bacterial strain | | MCC 852 | MCC 857 | MCC 859 | MCC 865 | MCC 866 | ATCC 19435 |
|---|---|---|---|---|---|---|---|---|
| XII | Growth temperature | 10°C | +S | +S | + | +S | +S | +S |
| | | 40°C | + | + | + | + | + | + |
| | | 45°C | - | - | - | - | - | - |
| XIII | alt resistance | 2% | + | + | + | + | + | + |
| | | 3% | + | + | + | + | + | + |
| | | 4% | + | + | + | + | + | + |
| | | 6.5% | (+)S | - | - | (+)S | (+)S | - |
| XIV | pH resistance | 9.2 | + | + | + | + | + | + |
| | | 9.6 | +S | + | + | + | - | - |
| XV | Methylene blue resistance | 0.01% | + | + | + | + | + | + |
| | | 0.1 % | + | + | + | + | + | + |
| | | 0.3 % | ± | +S | + | +S | + | - |
| XVI | Producibiliry of ammonia from arginine | | + | + | + | + | ± | + |
| XVII | Sugar fermentation | Arabinose | - | - | - | - | - | - |
| | | Xylose | - | - | - | - | - | + |
| | | Rhamnose | - | - | - | - | - | - |
| | | Ribose | + | + | +S | + | + | + |
| | | Glucose | + | + | + | + | + | + |
| | | Mannose | + | + | + | + | + | + |
| | | Fructose | + | + | + | + | + | + |
| | | Galactose | + | + | + | + | + | + |
| | | Sucrose | - | - | - | - | - | - |
| | | Maltose | + | + | + | + | + | + |
| | | Cellobiose | + | + | + | + | + | + |
| | | Lactose | + | + | + | + | + | + |
| | | Trehalose | + | + | + | + | + | + |
| | | Melibiose | - | - | - | - | - | - |
| | | Raffinose | - | - | - | - | - | - |
| | | Melezitose | - | - . | - | - | - | - |
| | | Dextrin | + | + | + | + | + | + |
| | | Starch | +S | + | - | + | + | ±S |
| | | Glycogen | - | - | - | - | - | - |
| | | Inulin | - | - | - | - | - | - |
| | | Mannitol | (+)S | + | + | - | - | - |
| | | Sorbitol | - | - | - | - | - | - |
| | | Inositol | - | - | - | - | - | - |
| | | Esculin | + | + | (+)S | + | + | +S |
| | | Salicin | + | + | +S | + | + | + |
| | | Amygdalin | - | ± | - | (+)S | (+)S | - |
| | | Methyl glucoside | - | - | - | - | - | - |
| | | Sodium gluconate | - | ± | + | - | - | - |
| +. Positive. (+): Slightly-positive. ±: Extremely slightly-positive. -: Negative. s: Slow reaction. | | | | | | | | |

The above-mentioned bacteriological properties (I) to (XI) are common to all of the 5 bacterial strains and the *Lactococcus lactis subsp. lactis-*type strain ATCC 19435. The growth temperature (XII), the salt-resistance (XIII), the pH-resistance (XIV), the methylene blue-resistance (XV), the producibility of ammonia from arginine (XVI), and the sugar fermentability (XVII) of each strain are shown in Table 1. The sugar fermentation was examined with respect to 28 kinds of sugar using a medium for sugar fermentation disclosed by Mitsuoka (Tomotari Mitsuoka, "The bacteriology of lactic acid bacteria", Clinical Examination 18, Pages 1163 to 1172, 1974).

It is apparent from the above-mentioned results that all of the 5 bacterial strains have the bacteriological properties common to *Lactococcus lactis subsp. lactis* bacterial strains. Thus, the 5 bacterial strains have been recognized to be bacterial strains belonging to *Lactococcus lactis subsp. lactis.* On the other hand, it is apparent from the above-mentioned bacteriological properties (XII) to (XVII) that the 5 bacterial strains are different from the *Lactococcus lactis subsp. lactis*-type strain in that the 5 bacterial strains do not have any abilities to ferment xylose.

The 5 bacterial strains were deposited by the applicant at the International Patent Organism Depositary, National Institute of Advanced Industrial Science and Technology, (Central 6, 1-1, Higashi 1-Chome Tsukuba-shi, Ibaraki-ken, JAPAN (postal code number: 305-8566)) as novel bacterial strains. The accession number of the *Lactococcus lactis subsp. lactis* MCC852 is FERM BP-10742, that of the *Lactococcus lactis subsp. lactis* MCC857 is FERM BP-10757, that of the *Lactococcus lactis subsp. lactis* MCC859 is FERM BP-10744, that of the *Lactococcus lactis subsp. lactis* MCC865 is FERM BP-10745, and that of the *Lactococcus lactis subsp. lactis* MCC866 is FERM BP-10746. The *Lactococcus lactis subsp. lactis* MCC852, 859, 865, and 866 were deposited on December 1, 2006, and the *Lactococcus lactis subsp. lactis* MCC857 was deposited on January 10, 2007.

### 3. Test with respect to fermentability on 10% (W/W) reconstituted skim milk medium.

Each 3% (V/V) bacterial strain starter was inoculated into a 10% (W/W) reconstituted skim milk medium sterilized at 95°C for 30 minutes, and then cultured at 25, 30, or 37°C for 16 hours. After the obtained culture medium was rapidly cooled, the curdled state was observed, and the pH and the viable count of the contained lactic acid bacteria were measured. The viable count was measured using commercially available BCP plate count agar (manufactured by Eiken Chemical Co., LTD.) flat plates. The measurement results are shown in Table 2.
*Lactococcus lactis subsp. lactis-type* strain ATCC 19435 disclosed in Patent Document 2 was used as a control strain.

**Table 2.**

| Bacterial strain | Culture condition | | | | | | | | |
|---|---|---|---|---|---|---|---|---|---|
| | at 25°C for 16 hours | | | at 30°C for 16 hours | | | at 37°C for 16 hours | | |
| | Viable count (CFUig) | pH | | Viable count (CFU/g) | pH | | Viable count (CFU/g) | pH | |
| MCC852 | 2.0×10⁸ | 4.53 | Curdled | 1.5×10⁹ | 4 44 | Curdled | 8.0×10⁸ | 4.63 | Curdled |
| MCC857 | 1.7 ×10⁹ | 4.53 | Curdled | 1.5 × 10⁹ | 4.41 | Curdled | 1.1 × 10⁹ | 4.5 | Curdled |
| MCC859 | 1.4×10⁹ | 4.54 | Curdled | 8.5×10⁸ | 4.44 | Curdled | 8.1×10⁸ | 4.59 | Curdled |
| MCC865 | 2.0×10⁹ | 4.52 | Curdled | 1.5×10⁹ | 4 42 | Curdled | 8.8×10⁸ | 4.63 | Curdled |
| MCC866 | 2.0×10⁹ | 4.52 | Curdled | 1.3×10⁹ | 4.4 | Curdled | 8.5×10⁸ | 4.61 | Curdled |
| ATCC 19435 | 5.2×10⁸ | 5.93 | Uncurdled | 4.4×10⁸ | 5.65 | Uncurdled | 3.2×10⁸ | 5.51 | Uncurdled |

When each bacterial strain of *Lactococcus lactis subsp. lactis* MCC852, 857, 859, 865, and 866, that is, the bacteria of the genus *Lactococcus* to be used in the present invention, was used, the pH of the culture medium was decreased to between 4.4 and 4.6 under any temperature conditions, and the culture medium was curdled. In addition, the viable count of the contained lactic acid bacteria was approximately 1×10⁹ CFU/g, and thus the favorable proliferation and fermentability conditions were recognized.
On the other hand, when the *Lactococcus lactis subsp. lactis-type* strain ATCC 19435 was used, the pH of the culture medium was 5.5 or more and the culture medium was not curdled under any temperature conditions. In addition, the viable count of the lactic acid bacteria was significantly less at 30°C or higher, than that particularly of the bacteria belonging to the genus *Lactococcus* according to the present invention.

### 4. Co-cultivation test with Bifidobacterium longum

### (1) Co-cultivation test with Bifidobacterium longum FERM BP-7787.

*Lactococcus lactis subsp. lactis*-type strain ATCC 19435 was used as a control strain.
First, each culture of the 5 bacterial strains (*Lactococcus lactis subsp. lactis* MCC852, 857, 859, 865, and 866) and *Bifidobacterium longum* FERM BP-7787 was prepared in accordance with the method described in the following Example 1.
In addition, 1,000 mL of a 10% (W/W) reconstituted skim milk medium containing 0.2% (W/W) yeast extract (manufactured by Difco) was sterilized at 90°C for 30 minutes. Then, 30 mL of a culture of the *Lactococcus lactis subsp. lactis-*type strain ATCC 19435 was inoculated into the reconstituted skim milk medium, and cultivated at 30°C for 16 hours to prepare a culture of the *Lactococcus lactis subsp. lactis-type* strain ATCC 19435.

1% (V/V) of each culture of the *Lactococcus lactis subsp. lactis* strains prepared as above was inoculated with 1% (V/V) of the culture of the *Bifidobacterium longum* FERM BP-7787 into a 10% (W/W) reconstituted skim milk medium sterilized at 90°C for 10 minutes, and the mixture were cultivated at 37°C for 16 hours to obtain fermented milk. The fermented milk was rapidly cooled, and the pH thereof and the viable count of the contained *Bifidobacterium* were measured. Then, the resultant was further stored at 10°C for two weeks, and the viable counts of the *Bifidobacterium* was measured at one week and two weeks after initiation of storage. The viable count of the *Bifidobacterium* was measured using TOS propionate agar (manufactured by YAKULT PHARMACEUTICAL INDUSTRY CO., LTD.) flat plates. The measurement results are shown in Table 3.

**Table 3.**

| Bacterial strain | Viable count *of Bifidobacterium* (CFU/g) | | | pH |
|---|---|---|---|---|
| | Immediately after end of fermentation | After one week storage | After two weeks storage | Immediately after end of fermentation |
| MCC852 | 5.7 × 10⁸ | 5.5 × 10⁸ | 5.5 × 10⁸ | 4.52 |
| MCC857 | 8.0 × 10⁸ | 7.5 × 10⁸ | 6.5 × 10⁸ | 4.47 |
| MCC859 | 6.8 × 10⁸ | 6.9 × 10⁸ | 5.7 × 10⁸ | 4.55 |
| MCC865 | 8.3 × 10⁸ | 8.0 × 10⁸ | 7.3 × 10⁸ | 4.56 |
| MCC866 | 6.4 × 10⁸ | 6.3 × 10⁸ | 5.3 × 10⁸ | 4.42 |
| ATCC19435 | 1.2 × 10⁸ | pH was 5 or more and storage test could not be performed. | | |

Each fermented milk prepared by using the *Lactococcus lactis subsp. lactis* MCC852, 857, 859, 865, or 866 had a pH of approximately 4.5 and a *Bifidobacterium* viable count of 5 × 10⁸ CFU/g or more after fermentation. When all the fermented milk was stored at 10°C for two weeks, the survival rate of *Bifidobacterium longum* was 80% or more.
On the other hand, fermentation of milk did not proceed with the *Lactococcus lactis subsp. lactis-*type strain ATCC 19435, and the pH of the fermented milk was 5.0 or more and storage thereof at 10°C was impossible. In addition, the *Bifidobacterium* viable count immediately after the end of fermentation was approximately 1 × 10⁸ CFU/g, which was significantly small in comparison with the case where the bacteria belonging to the genus *Lactococcus* according to the present invention were used.

Thus, it is apparent that the 5 bacterial strains (the *Lactococcus lactis subsp. lactis* MCC852, 857, 859, 865, and 866) are superior to other known bacterial strains of the *Lactococcus lactis subsp. lactis* in terms of the properties promoting the growth of *Bifidobacterium longum* FERM BP-7787 and the properties improving the survivability thereof during storage.
It is also apparent that in the case where *Bifidobacterium longum* is co-cultured with the *Lactococcus lactis subsp. lactis* that forms neither diacetyl nor acetoin as described in Patent Document 2 is unlike the case where *Bifidobacterium breve* is used, and neither the *Bifidobucterium-proliferation* promoting effects nor *Bifidobacterium-survivability* improving effects disclosed in Patent Document 2 are exhibited.

### (2) Co-cultivation test with Bifidobacterium longum-type strain ATCC 15707

The *Bifidobacterium longum* growth-promoting properties of the bacteria belonging to the genus *Lactococcus* to be used in the present invention and *Bifidobacterium longum* survivability-improving properties thereof during storage were checked using the *Bifidobacterium longum* FERM BP-7787 and the *Bifidobacterium longum-type* strain ATCC 15707.
First, a culture of the *Lactococcus lactis subsp. lactis* MCC857 and a culture of the *Bifidobacterium longum* FERM BP-7787 were prepared in accordance with the method described in the following Example 1.
In addition, a mixed culture of *Streptococcus thermophilus* and *Lactobacillus bulgaricus* was prepared in accordance with the method described in the following Example 2.
In addition, an 11% (W/W) skim milk medium containing 0.2% (W/W) yeast extract was sterilized at 90°C for 30 minutes. Then, 10% (V/V) of the *Bifidobacterium longum-*type strain ATCC 15707 was inoculated as a starter into the skim milk medium, and cultivated at 37°C until the pH reached 4.6 to prepare a culture of the *Bifidobacterium longum-*type strain ATCC 15707.

1% (V/V) of the culture of the *Lactococcus lactis subsp. lactis* MCC857 prepared as described above, either 1% (V/V) of the culture of the *Bifidobacterium longum* FERM BP-7787 or 1% (V/V) of the culture of the *Bifidobacterium longum-type* strain ATCC 15707, and 0.01% (V/V) of the mixed culture of *Streptococcus thermophilus* and *Lactobacillus bulgaricus* were inoculated into a 10% (W/W) reconstituted skim milk medium sterilized at 90°C for 10 minutes, and cultivated at 37°C until the pH reached 4.6 to obtain fermented milk. After the obtained fermented milk was rapidly cooled, the viable count of *Bifidobacterium* was measured. In addition, the fermented milk was stored at 10°C for two weeks, and the viable count of *Bifidobacterium longum* was measured at one week and two weeks after initiation of storage.
On the other hand, either 1.5% (V/V) of the culture of the *Bifidobacterium longum* FERM BP-7787 prepared as described above or 1.5% (V/V) of the culture of the *Bifidobacterium* longum-type strain ATCC 15707, and 0.4% (V/V) of the mixed culture of *Streptococcus thermophilus* and *lactobacillus bulgaricus* were inoculated into a 10% (W/W) reconstituted skim milk medium sterilized at 90°C for 10 minutes, and cultivated at 37°C until the pH reached 4.6 to obtain fermented milk as a control. The viable *Bifidobacterium* count in the fermented milk was measured in the same manner. The measurement results are shown in Table 4.

**Table 4.**

| MCC857 | *Bifidobacterium longum* | *Bifidobacterium* viable count (CFU/g) | | |
|---|---|---|---|---|
| | | Immediately after end of fermentation | After one week storage | After two weeks storage |
| Presence | FERMBP-7787 | 10 × 10⁹ | 1.0 × 10⁹ | 7.1 × 10⁸ |
| Presence | ATCC 15707 | 6.5 × 10⁸ | 3.8 × 10⁸ | 2.0 × 10⁸ |
| Absence | FERM BP-7787 | 2.0 × 10⁸ | 1.9 × 10⁸ | 4.0 × 10⁷ |
| Absence | ATCC 15707 | 3.0 × 10⁷ | 1.1 × 10⁶ | Undetectable |

Both viable counts of the *Bifidobacterium longum* FERM BP-7787 and the *Bifidobacterium longum-type* strain ATCC 15707 in fermented milk were significantly increased by co-cultivating with the *Lactococcus lactis subsp. lactis* MCC857. In addition, the survival rate of every *Bifidobacterium* stored at 10°C for two weeks was 30% or more: that of the *Bifidobacterium longum* FERM BP-7787 was 71% and that of the *Bifidobacterium longum-*type strain ATCC 15707 was 31%.
In contrast, the survival rate of the *Bifidobacterium longum* FERM BP-7787 stored at 10°C for two weeks after cultivating in the absence of *Lactococcus lactis subsp. lactis* MCC857 was 20% and no viable *Bifidobacterium longum-*type strain ATCC 15707 stored at 10°C for two weeks after cultivating in the absence of *Lactococcus lactis subsp. lactis* MCC857 was detected.
The same results were obtained when every *Lactococcus lactis subsp. lactis* MCC852, 859, 865, and 866 was used instead of the *Lactococcus lactis subsp. lactis* MCC857.

Thus, it is apparent that every *Lactococcus lactis subsp. lactis* MCC852, 857, 859, 865, and 866 also has excellent properties of both promoting the growth of *Bifidobacterium longum* strains other than *Bifidobacterium longum* FERM BP-7787 having an excellent survivability during storage and improving the survivability of *Bifidobacterium longum* strains other than *Bifidobacterium longum* FERM BP-7787 during storage.

### 5. Comparative test with mixture of Lactococcus lactis subsp. lactis and Lactococcus lactis subsp. cremoris disclosed in Patent Document 1.

The culture of *Lactococcus lactis subsp. lactis* MCC857, the culture of *Bifidobacterium longum*-type strain ATCC 15707, and the mixed culture of *Streptococcus thermophilus* and *Lactobacillus bulgaricus* were prepared in accordance with the method described in the above 4(2).

1% (V/V) of the culture of *Lactococcus lactis subsp. lactis* MCC857, 1% (V/V) of the culture of *Bifidobacterium longum-*type strain ATCC 15707, and 0.01% (V/V) of the mixed culture of *Streptococcus thermophilus* and *Lactobacillus bulgaricus,* prepared in the above manner, were inoculated into a 10% (W/W) reconstituted skim milk medium sterilized at 90°C for 10 minutes. The mixture was cultivated at 37°C until the pH reached 4.6 to prepare fermented milk. The fermented milk was rapidly cooled and the viable count of the contained *Bifidobacterium* was measured.
In contrast, 1% (V/V) of the culture of *Biidobacterium longum-*type strain ATCC 15707 prepared in the above-manner, and 2% (V/V) of a mixture "EZAL MA14" composed of *Lactococcus lactis subsp. lactis* and *Lactococcus lactis subsp. cremoris* (manufactured by Rhodia) were inoculated into a 10% (W/W) reconstituted skim milk medium sterilized at 90°C for 10 minutes, as a control. The mixture was cultivated at 38°C until the pH reached 4.6 to prepare fermented milk. The viable count of *Bifidobacterium* in the fennented milk was measured in the same manner. The mixture "EZAL MA14" corresponds to a mixture "EZAL MR014" (manufactured by Rhodia) described in Patent Document 1.

The viable *Bifidobacterium* count in fermented milk prepared using *Lactococcus lactis subsp. lactis* MCC857 was 5.5×10⁸ CFU/g. In contrast, no viable *Bifidobacterium* cells were detected in a diluted solution obtained by diluting fermented milk prepared using the mixture "EZAL MA14" by 10⁶ fold, and thus the viable *Bifidobacterium* count in the fermented milk was revealed to be 1 × 10⁶ CFU/g or less.

In other words, it was revealed that neither effects of promoting the growth of *Bifidobacterium* nor such effects of shortening fermentation time, as mentioned in Patent Document 1, were achieved when *Bifidobacterium longum* was co-cultivated with *Lactococcus lactis subsp. lactis* and *Lactococcus lactis subsp. cremoris.*

As described above, *Lactococcus lactis subsp. lactis* MCC852, 857, 859, 865, and 866 (the bacteria belonging to the genus *Lactococcus* to be used in the present invention) exhibit strong fermentability in the milk medium at a temperature suitable to fermentation with *Bifidobacterium,* and also exhibit excellent effects of promoting the growth of *Bifidobacterium* and improving the survivability thereof during storage, when co-cultivated with *Bifidobacterium longum.* Thus, it is apparent that the bacteria have properties which are not accompanied with conventionally known bacterial strains belonging to the genus *Lactococcus.* In addition, it is expected that the bacteria to be used in the present invention can produce fermented products with favorable taste, since the bacteria produce neither diacetyl nor acetoin.

Although a preculture medium used for cultivating *Bifidobacterium* and the bacteria belonging to the genus *Lactococcus* in advance is not particularly limited provided that the preculture medium is usually used, the preculture medium is preferably a milk medium. The preculture medium is more preferably a reconstituted skim milk medium, since the reconstituted skim milk medium can be easily handled. It is preferable that the concentration of the reconstituted skim milk medium be 3% (W/W) or more, and more preferably 8% (W/W) or more. In addition, the preculture medium may contain growth-stimulating substances such as yeast extract or reducing agents such as L-cysteine. It is particularly preferable that a growth-stimulating substance be formulated in the preculture medium, since *Bifidobacterium* exhibits a low level of proliferation in the milk medium. Specifically, a culture medium containing 0.1 to 1% (W/W) of yeast extract may be used. The preculture medium is subjected to sterilization for the use. The sterilization may be performed in accordance with a conventional method, specifically performed by heating at 80 to 122°C for 5 to 40 minutes, preferably at 85 to 95°C for 5 to 35 minutes.

The fermentation base medium to be used for fermentation with both *Bifidobacterium* and bacteria belonging to the genus *Lactococcus* is not particularly limited, provided that the fermentation base medium is usually used to produce fermented milk. The fermentation base medium may be prepared, for example, by formulating a sweetener such as sucrose, pectin, fruit, fruit juice, agar, gelatin, oil and fat, flavor, coloring agent, stabilizer, reducing agent, or the like, in cow's milk, skim milk, fresh cream, butter, whole milk powder, powdered skim milk, or the like, as needed, followed by sterilizing, homogenizing, cooling, and the like, in accordance with conventional methods.

Although the inoculation ratio of *Bifidobacterium* to the bacteria belonging to the genus *Lactococcus,* to be inoculated into the fermentation base medium as starters, is not particularly limited, the inoculation ratio is preferably 100:1 to 1:10, and more preferably 10:1 to 1:1. Although both amounts of *Bifidobacterium* and the bacteria belonging to the genus *Lactococcus* to be inoculated in the fermentation base medium are not particularly limited, it is preferable that the sum amount thereof be 0.01 to 10% (V/V), more preferably 0.1 to 5% (V/V), with respect to the amount of the fermentation base medium.

The lactic acid bacteria to be used in the present invention may further contain other lactic acid bacteria in addition to *Bifidobacterium* and the bacteria belonging to the genus *Lactococcus,* unless the effects of the bacteria belonging to the genus *Lactococuss* on the growth-stimulation of *Bifidobacterium* or the survivability-improvement thereof during storage are disturbed. Although the other lactic acid bacteria are not particularly limited, provided that the other lactic acid bacteria are usually used to produce fermented milk, the other lactic acid bacteria are preferably *Streptococcus thermophilus* and *Lactobacillus bulgaricus.* The inoculation ratio of the total content of *Bifidobacterium* and the bacteria belonging to the genus *Lactococcus* to the total content of the other lactic acid bacteria, to be inoculated as starters into a fermentation base medium, is not particularly limited, unless the effects of the bacteria belonging to the genus *Lactococcus* on *Bifidobacterium* are disturbed, it is preferable that the inoculation ratio be within a range of 1,000:1 to 10:1.

It is preferable that co-cultivation temperature in the method for producing fermented milk according to the present invention be within a range of 30°C to 40°C, more preferably 36°C to 38°C. Both *Bifidobacterium* and the bacteria belonging to the genus *Lactococcus* to be used in the present invention can be sufficiently proliferate in the above-mentioned temperature range. Although the cultivation time period is suitably determined depending on the kind of fermented milk to be prepared, the cultivation time period is preferably within a range of 5 to 18 hours.

The fermented milk obtained by cultivation may be provided as a food product, or may be homogeneously processed into a liquid state. In addition, fruit juice, fruit, or the like, may be suitably formulated in the fermented milk. The fermented milk may be put into a container by a conventionally-used method without any particular limitations.

In the following, the present invention will be circumstantially explained by indicating some examples. However, the present invention is not limited to the following examples.

### EXAMPLE 1

1000 mL of 10% (W/W) reconstituted skim milk medium prepared by dissolving 10% by mass of skim milk powders (manufactured by Morinaga Milk Industry Co., Ltd.) in water was sterilized at 90°C for 30 minutes, and then 30 mL of a seed culture of *Lactococcus lactis subsp. lactis* MCC857 was inoculated thereinto, followed by cultivating at 25°C for 16 hours. On the other hand, 1000 ml of an 11% (W/W) skim milk medium containing 0.2% (W/W) yeast extract was sterilized at 90°C for 30 minutes, and 100 mL of a seed culture of *Bifidobacterium longum* FERM BP-7787 was inoculated thereinto, followed by cultivating at 37°C for 6 hours.
A part from the above, 50L of a base medium prepared by mixing and dissolving raw materials composed of skim milk powders, whole milk powders, pectin, and sucrose, the base medium containing 0.5% (W/W) of butterfat, 8.0% (W/W) of nonfat milk solid component, 5.0% (W/W) of sucrose, and 0.2% (W/W) of pectin, was sterilized at 90°C for 10 minutes, followed by cooling at 40°C. 50 mL of the above-obtained culture of the *Lactococcus lactis subsp. lactis* MCC857 precultured and 500 mL of the above-obtained culture of *Bifidobacterium longum* FERM BP-7787 precultured were inoculated into the sterilized base medium, followed by cultivating at 37°C for 16 hours to obtain fermented milk. The fermented milk was immediately cooled while stirring, and the cooled fermented milk was homogenized at a pressure of 15 MPa, followed by putting the resultant into a glass container having a 200 mL capacity and then sealing the container to obtain a yogurt drink. The obtained yogurt drink had a lactic acid content of 0.68%, a pH of 4.64, and a viscosity of 75 mPa· s, and contained 7.0×10⁸ CFU/g of *Bifidobacterium.* When the yogurt drink was stored at 10°C for 21 days, the viable count of *Bifidobacterium* was 5.2 × 10⁸ CFU/g, and the survival rate thereof was 74%.

### EXAMPLE 2

30 mL of a seed culture of *Lactococcus lactis subsp. lactis* MCC866 was inoculated into 1000 mL of a 10% (W/W) reconstituted skim milk medium sterilized at 90°C for 30 minutes, and then cultivated at 25°C for 16 hours. On the other hand, 1000 mL of an 11% (W/W) skim milk medium containing 0.2% (W/W) yeast extract was sterilized at 90°C for 30 minutes, and 100 ml of a seed culture of *Bifidobacterium longum* FERM BP-7787 was inoculated thereinto, followed by cultivating at 37°C for 6 hours. Apart form the above, 50 mL of a mixed culture of *Streptococcus thermophilus* (manufactured by HANSEN) and *Lactobacillus bulgaricus* (manufactured by HANSEN) was inoculated into 1500 ml of a 10% (W/W) reconstituted skim milk medium sterilized at 90°C for 30 minutes, and then cultivated at 37°C for 5 hours.
Apart from the above, 50 L of raw milk containing 3.0% (W/W) of butterfat and 9.0% (W/W) of nonfat milk solid component were heated at 70°C, homogenized at a pressure of 15 MPa, sterilized at 90°C for 10 minutes, and then cooled at 40°C. Into the thus sterilized base medium, 500 mL of the culture of *Lactococcus lactis subsp. lactis* MCC866 precultured as described above, 500 mL of the culture of *Bifidobacterium longum* FERM BP-7787, and 5 ml of the mixed culture of *Streptococcus thermophilus* and *Lactobacillus bulgaricus* were inoculated. The resultant was put into a resin container having a 500 mL capacity, and then the container was sealed. The bacteria were cultivated at 37°C for 7 hours, and then immediately cooled. The thus obtained fermented milk had a lactic acid content of 0.72%, and a pH of 4.55, and contained *5.8 × 10⁸ CFU*/*g of Bifidobacterium.* When the fermented milk was stored at 10°C for 21 days, the viable count of *Bifidobacterium* was 3.6×10⁸ CFU/g, and the survival rate thereof was 62%.

### Example 3

30 mL of a seed culture of *Lactococcus lactis subsp. lactis* MCC865 was inoculated into 1000 mL of a 10% (W/W) reconstituted skim milk medium sterilized at 90°C for 30 minutes, and then cultivated at 25°C for 16 hours. On the other hand, 1000 mL of an 11% (W/W) skim milk medium containing 0.2% (W/W) yeast extract was sterilized at 90°C for 30 minutes, and 100 ml of a seed culture of *Bifidobacterium longum* FERM BP-7787 was inoculated thereinto, followed by cultivation at 37°C for 6 hours. Apart from the above, 50 mL of a mixed culture of *Streptococcus thermophilus* (manufactured by HANSEN) and *Lactobacillus bulgaricus* (manufactured by HANSEN) was inoculated into 1500 ml of a 10% (W/W) reconstituted skim milk medium sterilized at 90°C for 30 minutes, and then cultivated at 42°C for 5 hours.
Apart from the above, 500 L of raw milk containing 3.4% (W/W) of butterfat and 12.2% (W/W) of nonfat milk solid component were heated at 70°C, homogenized at a pressure of 15 MPa, sterilized at 90°C for 10 minutes, and then cooled at 40°C. Into the thus sterilized base medium, 5 L of the culture of *Lactococcus lactis subsp. lactis* MCC865 precultured as described above, 5 L of the culture of *Bifidobacterium longum* FERM BP-7787, and 50 ml of the mixed culture of *Streptococcus thermophilus* and *Lactobacillus bulgaricus* were inoculated, and then cultivated at 37°C for 7.5 hours, followed by immediately cooling. The thus obtained fermented milk was further stirred to pulverize curd, and the pulverized fermented milk was mixed with blueberry preserve prepared by a conventional method at a ratio of 7:3. The mixture was homogenized by stirring, and then put into an oxidation barrier paper-container having a 120 mL capacity. The thus obtained fermented milk with blueberry pulp had a lactic acid content of 0.80%, and a pH of 4.45, and contained 5.5×10⁸ CFU/g of *Bifidobacterium.* When the fermented milk with blueberry pulp was stored at 10°C for 21 days, the viable count of *Bifidobacterium* was 3.9×10⁸ CFU/g, and the survival rate thereof was 71%.

### INDUSTRIAL APPLICABILITY

Since fermented milk containing a greater count of viable *Bifidobacterium,* than ever before, even immediately before the end of use-by-date, can be produced in accordance with the production method according to the present invention, the production method is available in the field of production of fermented milk or the like.

## Claims

1. A method for producing a fermented milk, comprising:
performing fermentation using both bacteria belonging to a genus *Bifidobacterium* and bacteria belonging to a genus *Lactococcus* as lactic acid bacteria,
wherein the bacteria belonging to the genus *Lactococcus* have bacteriological properties of:
(1) a fermentability which curdles a 10% (W/W) reconstituted skim milk medium when cultivated at a temperature of 25°C to 37°C for 16 hours;
(2) *Bifidobacterium longum* growth-promoting properties which lead a viable count of *Bifidobacterium longum* of 5 × 10⁸ CFU/g or more, when co-cultivated with *Bifidobacterium longum* in the 10% (W/W) reconstituted skim milk medium until a pH thereof is 4.4 to 4.6; and
(3) *Bifidobacterium longum* survivability-improving properties during storage, which lead to a survival rate of *Bifidobacterium longum* of 30% or more, after co-cultivation with *Bifidobacterium longum* in the 10% (W/W) reconstituted skim milk medium until the pH thereof is 4.4 to 4.6, rapid cooling, and two weeks storage at 10°C.

2. The method for producing a fermented milk according to Claim 1, wherein the bacteria belonging to the genus *Lactococcus* have no ability to ferment xylose and produce neither diacetyl nor acetoin.

3. The method for producing a fermented milk according to Claim 1 or 2, wherein the bacteria belonging to the genus *Lactococcus* are *Lactococcus lactis* subsp. *lactis.*

4. The method for producing a fermented milk according to any one of Claims 1 to 3, wherein the bacteria belonging to the genus *Lactococcus* comprise at least one bacterial strain selected from the group consisting of *Lactococcus lactis subsp, lactis* MCC852 (FERM BP-10742), *Lactococcus lactis subsp. lactis* MCC857 (FERM BP-10757), *Lactococcus lactis subsp. lactis* MCC859 (FERM BP-10744), *Lactococcus lactis subsp. lactis* MCC865 (FERM BP-10745), and *Lactococcus lactis subsp. lactis* MCC866 (FERM BP-10746).

5. The method for producing a fermented milk according to any one of Claims 1 to 4, wherein the bacteria belonging to the genus *Bifidobacterium* are *Bifidobacterium longum.*

6. The method for producing a fermented milk according to Claim 5, wherein a bacterial strain of the *Bifidobacterium longum* is *Bifidobacterium longum* FERM BP-7787.

7. The method for producing a fermented milk according to any one of Claims 1 to 6, wherein both *Streptococcus thermophilus* and *Lactobacillus bulgaricus* are further used as the lactic acid bacteria.

8. A fermented milk prepared by the method for producing fermented milk of any one of Claims 1 to 7.

## Patentansprüche

1. Verfahren zum Herstellen einer fermentierten Milch, umfassend:
Durchführen einer Fermentation unter Verwendung sowohl von Bakterien, die zu einer Gattung *Bifidobacterium* gehören, als auch von Bakterien, die zu einer Gattung *Lactococcus* gehören, als Milchsäurebakterien,
wobei die zur Gattung *Lactococcus* gehörenden Bakterien folgende bakteriologische Eigenschaften aufweisen:
(1) eine Gärfähigkeit, die ein 10%iges (Gew./Gew) rekonstituiertes Magermilchmedium zum Gerinnen bringt, wenn es bei einer Temperatur von 25°C bis 37°C 16 Stunden lang kultiviert wird;
(2) das Wachstum von *Bifidobacterium longum* fördernde Eigenschaften, die zu einer Lebendzahl von *Bifidobacterium longum* in Höhe von 5 x 10⁸ CFU/g oder mehr führen, wenn sie mit *Bifidobacterium longum* in dem 10%igen (Gew./Gew) rekonstituierten Magermilchmedium kokultiviert werden, bis ein pH-Wert davon 4,4 bis 4,6 beträgt, und
(3) die Überlebensfähigkeit von *Bifidobacterium longum* verbessernde Eigenschaften während der Aufbewahrung, die zu einer Überlebensrate von *Bifidobacterium longum* in Höhe von 30% oder mehr führen, nach Kokultivierung mit *Bifidobacterium longum* in dem 10%igen (Gew./Gew) rekonstituierten Magermilchmedium, bis der pH-Wert davon 4,4 bis 4,6 beträgt, schnellem Abkühlen und einer Aufbewahrung von zwei Wochen bei 10°C.

2. Verfahren zum Herstellen einer fermentierten Milch gemäß Anspruch 1, wobei die zur Gattung *Lactococcus* gehörenden Bakterien keine Fähigkeit aufweisen, Xylose zu fermentieren und weder Diacetyl noch Acetoin produzieren.

3. Verfahren zum Herstellen einer fermentierten Milch gemäß Anspruch 1 oder 2, wobei es sich bei den zur Gattung *Lactococcus* gehörenden Bakterien um *Lactococcus lactis* subsp. *lactis* handelt.

4. Verfahren zum Herstellen einer fermentierten Milch gemäß einem der Ansprüche 1 bis 3, wobei die zur Gattung *Lactococcus* gehörenden Bakterien mindestens einen Bakterienstamm, ausgewählt aus der Gruppe, bestehend aus *Lactococcus lactis subsp. lactis* MCC852 (FERM BP-10742), *Lactococcus lactis subsp. lactis* MCC857 (FERM BP-10757), *Lactococcus lactis subsp. lactis* MCC859 (FERM BP-10744), *Lactococcus lactis subsp. lactis* MCC865 (FERM BP-1 0745) und *Lactococcus lactis subsp. lactis* MCC866 (FERM BP-10746), umfassen.

5. Verfahren zum Herstellen einer fermentierten Milch gemäß einem der Ansprüche 1 bis 4, wobei es sich bei den zur Gattung *Bifrdobacterium* gehörenden Bakterien um *Bifidobacterium longum* handelt.

6. Verfahren zum Herstellen einer fermentierten Milch gemäß Anspruch 5, wobei es sich bei einem Bakterienstamm von *Bifidobacterium longum* um *Bifidobacterium longum* FERM BP-7787 handelt.

7. Verfahren zum Herstellen einer fermentierten Milch gemäß einem der Ansprüche 1 bis 6, wobei ferner sowohl *Streptococcus thermophilus* als auch *Lactobacillus bulgaricus* als die Milchsäurebakterien verwendet werden.

8. Fermentierte Milch, hergestellt durch das Verfahren zum Herstellen fermentierter Milch nach einem der Ansprüche 1 bis 7.

## Revendications

1. Procédé pour produire un lait fermenté, comprenant :
la réalisation d'une fermentation en utilisant à la fois des bactéries appartenant au genre *Bifidobacterium* et des bactéries appartenant au genre *Lactococcus* en tant que bactéries lactiques,
dans lequel les bactéries appartenant au genre *Lactococcus* ont les propriétés bactériologiques consistant en :
(1) une fermentabilité qui caille un milieu à base de lait écrémé reconstitué à 10% (masse/masse) lorsque cultivées à une température de 255°C à 37°C pendant 16 heures ;
(2) des propriétés favorisant la croissance de *Bifidobacterium longue* ce qui entraîne un titre viable en *Bifidobacterium longum* de 5×10⁸ CFU/g ou plus, lorsque co-cultivées avec des *Bifidobacterium longum* dans le milieu à base de lait écrémé reconstitué à 10% (masse/masse) jusqu'à ce que le pH de celui-ci soit de 4,4 à 4,6 ; et
(3) des propriétés améliorant la capacité de survie de *Bifidobacterium longue* durant le stockage, ce qui entraîne un taux de survie des *Bifidobacterium longum* de 30% ou plus, après une co-culture avec des *Bifidobacterium longum* dans le milieu à base de lait écrémé reconstitué à 10% (masse/masse) jusqu'à ce que le pH de celui-ci soit de 4,4 à 4,6, un rapide refroidissement et un stockage de deux semaines à 10°C.

2. Procédé pour produire un lait fermenté selon la revendication 1, dans lequel les bactéries appartenant au genre *Lactococcus* n'ont pas la capacité à fermenter le xylose et ne produisent ni du diacétyle ni de l'acétoïne.

3. Procédé pour produire un lait fermenté selon la revendication 1 ou 2, dans lequel les bactéries appartenant au genre *Lactococcus* sont des *Lactococcus lactis* ssp. *lactis.*

4. Procédé pour produire un lait fermenté selon l'une quelconque des revendications 1 à 3, dans lequel les bactéries appartenant au genre *Lactococcus* comprennent au moins une souche bactérienne choisie dans le groupe constitué par *Lactococcus lactis ssp. lactis* MCC852 (FERM BP-10742), *Lactococcus lactis ssp. lactis* MCC857 (FERM BP-10757), *Lactococcus lactis ssp. lactis* MCC859 (FERM BP-10744), *Lactococcus lactis ssp. lactis* MCC865 (FERM BP-10745), et *Lactococcus lactis ssp. lactis* MCC866 (FERM BP-10746).

5. Procédé pour produire un lait fermenté selon l'une quelconque des revendications 1 à 4, dans lequel les bactéries appartenant au genre *Bifidobacterium* sont des *Bifidobacterium longum.*

6. Procédé pour produire un lait fermenté selon la revendication 5, dans lequel la souche bactérienne de *Bifidobacterium longum* est la *Bifidobacterium longum* FERM BP-7787.

7. Procédé pour produire un lait fermenté selon l'une quelconque des revendications 1 à 6, dans lequel à la fois des *Streptococcus thermophilus* et des *Lactobacillus bulgaricus* sont en outre utilisées en tant que bactéries lactiques.

8. Lait fermenté préparé par un procédé pour produire du lait fermenté selon l'une quelconque des revendications 1 à 7.
